# EUROPÄISCHE PATENTSCHRIFT

(11) **EP 3 672 680 B1**
(45) Veröffentlichungstag und Bekanntmachung des Hinweises auf die Patenterteilung: **20.04.2022**
(21) Anmeldenummer: 18734794.3
(22) Anmeldetag: 26.06.2018
(51) Int. Cl.: A61M 37/00

(54) **VORRICHTUNG ZUM IMPLANTIEREN DER OBJEKTE UNTERHALB DER HAUT**
DEVICE FOR IMPLANTING OBJECTS UNDER SKIN
DISPOSITIF SERVANT A IMPLANTER DES OBJETS SOUS LA PEAU

(30) Priorität: 23.08.2017 DE 202017105048 U
(43) Veröffentlichungstag der Anmeldung: 01.07.2020
(73) Patentinhaber: H&B Electronic GmbH & Co. KG, 75392 Deckenpfronn (DE)
(72) Erfinder: MORLOK, Tobias, 71159 Mötzingen (DE); PETRY, Dariusz, 71134 Aidlingen (DE)
(74) Vertreter: Wacker, Jost Oliver
(86) Internationale Anmeldenummer: PCT/EP2018/067123
(87) Internationale Veröffentlichungsnummer: WO 2019/037925

(56) Entgegenhaltungen:
- WO-A1-2006/077250
- US-A- 4 994 028
- US-A1- 2004 199 140
- US-B1- 6 270 472

## Beschreibung

Die Erfindung betrifft eine Implantiervorrichtung zum Setzen mindestens eines Objektes unterhalb der Haut eines Patienten nach dem Oberbegriff des Anspruchs 1. Diese Implantiervorrichtung weist ein Gehäuse sowie einen Kanülenhalter auf, der gegenüber dem Gehäuse verlagerbar gehalten ist und an dem eine Kanüle anbringbar beziehungsweise angebracht ist, die eine distale Spitze und einen Hohlraum für die Aufnahme des mindestens einen zu implantierenden Objektes aufweist. Ferner weist die Implantiervorrichtung einen gegenüber dem Gehäuse festlegbaren beziehungsweise festgelegten Stößel auf, der ein distales Ende bildet, das zum Abstützen der zu implantierenden Objekte in proximaler Richtung im Hohlraum der Kanüle positioniert werden kann. Dabei kann die Kanüle von einer ersten Einstechstellung gegenüber einem Gehäuseaustritt über einen ersten Rückhubweg entlang des Stößels in eine erste Einrückstellung verlagert werden, um dadurch ein im Hohlraum aufgenommenes erstes Objekt abzulegen. Dabei ist die Kanüle von der ersten Einrückstellung gemeinsam mit dem Stößel, das heißt positionsstabil gegenüber dem Stößel, in eine zweite Einstechstellung gegenüber dem Gehäuseaustritt verlagerbar. Zudem kann die Kanüle dabei von der zweiten Einstechstellung über einen zweiten Rückhubweg entlang des Stößels in eine zweite Einrückstellung verlagert werden, um ein im Hohlraum aufgenommenes zweites Objekt aus der Kanüle heraus abzulegen beziehungsweise zu setzen. Auf diese Weise kann die Kanüle zusammen mit dem Stößel in eine Position verbracht werden, aus der heraus wenigstens eine zweite vordefinierte Setzbewegung zum Ablegen eines weiteren Objekts ausgeführt werden kann. Hierdurch ist die Implantiervorrichtung nachladbar beziehungsweise aufziehbar, um wenigstens zwei in der Kanüle aufgenommene Implantate hintereinander mittels einer vorbestimmten Relativbewegung der Kanüle gegenüber dem Stößel sicher setzen zu können.

Derartige Implantiervorrichtungen ermöglichen das Setzen von insbesondere länglichen Implantaten, die beispielsweise zur Langzeitabgabe von medizinischen Wirkstoffen oder Hormonen im Körper eines Menschen oder eines Tieres dienen. Alternativ hierzu können die Implantate auch durch elektronische Bauteile gebildet sein, die beispielsweise zur Identifikation des Trägers oder zur Energieversorgung von medizinischen Implantaten dienen.

DE3880676T2 zeigt eine Implantiervorrichtung zum fächerförmigen Anordnen zweier Arzneimittel-Implantate unter der Haut eines Patienten. Die beiden Implantate werden hierzu in einer Kanüle hintereinander angeordnet, die an einer Nabe gehalten ist. Zum Setzen des ersten Implantates wird die Nabe mit der Kanüle entlang eines Stößels verschoben und anschließend zur Vorbereitung des zweiten Setzvorganges wieder in die entgegen gesetzte Richtung bewegt. Hierbei wird ein teleskopierbarer Teil des Stößels ausgefahren, an dem das zweite Implantat während des zweiten Setzvorganges rückseitig abgestützt ist.

DE69731843T2 beschreibt eine Implatiervorrichtung zum Setzen mehrerer Feststoffkörper. Die Feststoffkörper sind hierzu hintereinander in einer Kanüle angeordnet, wobei sich der hinterste Feststoffkörper rückseitig an einem entlang der Kanüle verlagerbaren Stößel abstützt. An dem Stößel sind hierbei Rastmittel vorgesehen, die bei Verlagerung der Kanüle dem Benutzer geeignete Stellungen des Stößels zum Setzen der einzelnen Feststoffkörper anzeigen.

US6,270,472B1 beschreibt eine Vorrichtung zum Setzen mehrerer kurzer Implantate in einen Tumor, wobei die Implantate beispielsweise ein radioaktives Isotop enthalten können. Die Vorrichtung weist zwei zueinander verlagerbare Gehäuseteile auf, wobei am inneren Gehäuseteil eine Kanüle angeschraubt ist, in der die Implantate aufgenommen sind, und durch die ein Stößel zum Auswerfen der Implantate ragt. Der Stößel ist zum nacheinander erfolgenden Auswurf mehrerer Implantate über einen Betätigungsmechanismus wiederkehrend in verschiedenen Positionen gegenüber dem inneren Gehäuseteil festlegbar beziehungsweise mittels eines innerhalb des inneren Gehäuses verschiebbaren Schlittens in die Kanüle hinein verlagerbar.

US2003/0040699A1 zeigt eine Implantiervorrichtung für die Durchführung eines einzelnen Setzvorganges, bei dem eine mit Implantaten gefüllte Kanüle durch Betätigung eines Schiebeknopfes entlang eines Stößels bewegt wird, wodurch die Implantate freigesetzt werden.

US2004/0199140 zeigt ebenso wie US2008/0221510A1 und US2009/0012463A1 eine Implantiervorrichtung mit einem am Gehäuse fest gelagerten Stößel, bei der die Kanüle mit einem entlang einer geschwungenen Führung verlagerbaren Schiebeknopf verbunden ist. Über den Schiebeknopf kann die Kanüle dabei jeweils entlang des Stößels verschoben werden, um die in der Kanüle aufgenommenen Implantate freizusetzen.

Aus WO 2006/077250 A1 ist eine Implantiervorrichtung zum Setzen von länglichen Implantaten unter die Haut von Menschen oder Tieren bekannt. Diese weist eine aus einem Gehäuse herausragende Kanüle auf, in der sich das Implantat an einem proximalen Ende an einem Stößel abstützt, der im Gehäuse positionsstabil gehalten ist. Nach dem Einstechen und Positionieren eines distalen Abschnittes der Kanüle unter der Haut des Patienten, wird die Kanüle mittels einer verschiebbaren Handhabe in proximaler Richtung des Gehäuses verschoben, wobei das Implantat unter die Haut des Patienten gesetzt wird.

Nachteilig an der bekannten Implantiervorrichtung ist, dass mit ihr nur eine einzige in ihrer Länge vordefinierte Setzbewegung ausgeführt werden kann, weshalb jeweils nur ein sicherer Setzvorgang zur Positionierung eines länglichen Implantats unter der Haut eines Patienten durchgeführt werden kann. Bevor ein zweites Objekt mit der gleichen Implantiervorrichtung gesetzt werden kann, muss dagegen sowohl die Kanüle als auch der Stößel ersetzt oder zumindest gereinigt sowie das weitere Objekt neu eingesetzt werden. Darüber hinaus kann die Implantiervorrichtung aufgrund des relativ langen Betätigungsweges der Handhabe in der Regel nur mit zwei Händen bedient werden.

Die Aufgabe der Erfindung ist es, bei einer gattungsgemäßen Implantiervorrichtung die genannten Nachteile zu vermeiden und sowohl ein komfortables als auch störungsfreies Implantieren, insbesondere von zwei oder mehreren Objekten zu ermöglichen.

Diese Aufgabe wird durch eine Implantiervorrichtung mit den Merkmalen des Anspruchs 1 gelöst. Dabei kann der Kanülenhalter zur Verlagerung in die zweite Einstechstellung an einem ersten Gehäuseteil festgelegt werden, das mit dem distalen Ende des Stößels fest verbunden ist. Dieses erste Gehäuseteil ist hierbei an einem zweiten Gehäuseteil verlagerbar gehalten, das wiederum den Gehäuseaustritt bildet. Hierdurch kann das Nachladen der Implantiervorrichtung für den zweiten Setzvorgang durch eine einfach auszuführende Relativbewegung zwischen den beiden Gehäuseteilen erfolgen, was eine besonders komfortable Durchführung des wenigstens einen weiteren Setzvorganges gewährleistet.

Dabei ist es günstig, wenn das erste Gehäuseteil verschiebbar am zweiten Gehäuseteil gelagert ist, um eine störungsfreie und exakte Relativbewegung zwischen beiden Gehäuseteilen zu gewährleisten.

Vorteilhafterweise weist das zweite Gehäuseteil an einer Außenseite rutschhemmende Mittel auf. Hierdurch ist es bereits durch leichtes Andrücken des zweiten Gehäuseteils an der Haut des betreffenden Patienten möglich, dieses positionsstabil zu halten, während das erste Gehäuseteil relativ dazu verlagert wird. Auf diese Weise kann die Implantiervorrichtung nach dem Setzen des ersten Implantats mit nur einer Hand in die zweite Einstichsstellung verbracht werden, aus der heraus der zweite Setzvorgang ausgeführt werden kann.

Zudem ist es günstig, wenn zwischen dem ersten Gehäuseteil und dem zweiten Gehäuseteil Gehäusearretiermittel vorgesehen sind, die bis zum Erreichen der ersten Einrückstellung der Kanüle beide Gehäuseteile sicher aneinander festlegen. Hierdurch kann sichergestellt werden, dass es während des ersten Setzvorganges zu keiner störenden Relativbewegung zwischen beiden Gehäuseteilen kommt.

In einer besonders vorteilhaften Ausführungsform der Implantiervorrichtung sind Rückholmittel vorgesehen, mittels denen die Verlagerung der Kanüle über wenigstens einen der Rückhubwege hinweg selbsttätig ausgeführt werden kann. Auf diese Weise kann die Verlagerung der Kanüle zum Setzen des betreffenden Implantats selbsttätig erfolgen, was eine besonders komfortable Handhabung der Implantiervorrichtung ermöglicht. Hierdurch ist insbesondere eine komfortable Einhandbedienung der Implantiervorrichtung möglich. Ferner ist es günstig, wenn die Rückholmittel einen mechanischen Kraftspeicher, aufweisen, mittels dem der Kanülenhalter beziehungsweise die Kanüle über den betreffenden Rückhubweg hinweg verlagert werden kann. Hierzu kann der Kraftspeicher beispielsweise über eine Rollfeder mit dem Kanülenhalter verbunden sein. Hierdurch können die für die selbsttätige Rückholbewegung benötigten Rückholmittel auf besonders einfache und kostengünstige Weise zur Verfügung gestellt werden.

In einer weiteren vorteilhaften Ausführungsform weisen die Rückholmittel ein Getriebe auf, über das der Kanülenhalter verlagert werden kann. Auf diese Weise kann eine bevorzugte Übersetzung zwischen der Bewegung des mechanischen Kraftspeicher und des Kanülenhalters eingestellt werden.

Ferner ist es günstig, wenn die Rückholmittel eine Dämpfungseinrichtung aufweisen, mittels der eine Verlagerungsgeschwindigkeit während der Bewegung über wenigstens einen der Rückhubwege hinweg eingestellt werden kann. Hierdurch kann die Verlagerungsgeschwindigkeit der Kanüle während ihrer Bewegung über den Rückhubweg hinweg derart begrenzt werden, dass im Implantierbereich eine minimierte Beeinträchtigung des Gewebes gewährleistet werden kann.

Dabei ist es vorteilhaft, wenn die Rückholmittel einen Auslöseknopf aufweisen, mittels dem die Verlagerung über wenigstens einen der Rückhubwege hinweg ausgelöst werden kann. Der Auslöseknopf kann dabei insbesondere derart ausgestaltet sein, dass nach einer Verlagerung über den vorgegebenen Rückhubweg hinweg unabhängig von der Stellung des Auslöseknopfes ein automatischer Stopp erfolgt. Zudem kann vorgesehen sein, dass der Auslöseknopf nach Durchlaufen des ersten Rückhubweges in erneuten Eingriff mit dem Kanülenhalter kommt, der nach nochmaligem Betätigen des Auslöseknopfes über einen zweiten Rückhubweg verlagert wird. In jedem Fall ermöglicht ein derartiger Auslöseknopf eine besonders komfortable Bedienung der Implantiervorrichtung mit nur einer Hand.

Zudem ist es dabei günstig, wenn an dem Auslöseknopf Festlegemittel vorgesehen sind die mit Festlegegegenmitteln des Kanülenhalters lösbar verbunden werden können. Hierdurch ist es möglich, den Auslöseknopf trotz gleichbleibender Position am Gehäuse für wenigstens zwei nacheinander vorzunehmende Setzvorgänge verwenden zu können.

In einer alternativen Ausführungsform der Implantiervorrichtung kann die Verlagerung der Kanüle beziehungsweise des Kanülenhalters über wenigstens einen der Rückhubwege manuell ausgeführt werden, wodurch die implantierende Person die Bewegung der Kanüle über den betreffenden Rückhubweg hinweg besonders gut kontrollieren kann.

Hierzu ist es vorteilhaft, wenn zur manuellen Verlagerung der Kanüle beziehungsweise des Kanülenhalters ein am Gehäuse verschiebbarer Schiebeknopf vorgesehen ist, der durch Kraftbeaufschlagung aus einer Arretierung gelöst werden kann. Durch diese lösbare Arretierung kann dabei eine versehentliche Verlagerung des Schiebeknopfes und eine damit einhergehende unbeabsichtigte Betätigung der Kanüle vermieden werden.

Vorteilhafterweise ist die Kanüle in einer letzten Einrückstellung, die beispielsweise der zweiten Einrückstellung entspricht, vollständig innerhalb des Gehäuses aufgenommen. Auf diese Weise können nach Abschluss aller vorgesehenen Setzvorgänge Verletzungen durch die aus dem Gehäuse heraus ragende Kanüle verhindert werden. Hierbei ist es günstig, wenn eine Endlagensicherung vorgesehen ist, mittels der der Kanülenhalter in der besagten letzten Einrückstellung gegenüber dem Gehäuse festgelegt werden kann, sodass eine versehentliche Verlagerung des Kanülenhalters beziehungsweise der Kanüle aus dem Gehäuseaustritt heraus vermieden werden kann.

Vorteilhafterweise kann der Stößel dabei in der zweiten Einrückstellung vom Gehäuse getrennt werden. Hierdurch ist es beispielsweise möglich, nach Abschluss der vorgenommenen Setzvorgänge die Kanüle und gegebenenfalls auch den Stößel von der Implantiervorrichtung zu trennen und zu entsorgen.

In einer besonders bevorzugten Ausführungsform weisen die Kanüle und der Stößel jeweils einen von einem Kreisprofil abweichenden Querschnitt auf, der beispielsweise mehreckig, oval, nierenförmig, elliptisch oder ähnlich ausgeführt sein kann. Hierdurch ist es möglich, ein entsprechend geformtes Objekt in einer vorbestimmten Drehposition bezüglich der Längsachse der Kanüle aufzunehmen, während des Setzvorganges entlang der Kanüle zu führen und entsprechend unter der Haut des Patienten zu positionieren. Auf diese Weise kann das Implantat eine Position einnehmen, die hinsichtlich einer bestimmten Funktion des Implantats besonders vorteilhaft ist. Beispielsweise kann eine lichtempfindliche beziehungsweise eine Lichtenergie nutzende Seite des Implantats der Hautoberfläche zugewandt ausgerichtet werden, um eine optimale Energieerzeugung zu gewährleisten.

Vorteilhafterweise bildet das Gehäuse an einem distalen Ende einen Anschlag aus, der zur Begrenzung einer Einstichtiefe dient. Durch einen derartigen Anschlag ist es für den Benutzer der Implantiervorrichtung in besonders einfacher Weise möglich, eine für den implantieren Vorgang geeignete Einstichtiefe der Kanüle hinsichtlich der Haut des Patienten einzuhalten.

Dabei ist es günstig, wenn an dem Anschlag eine Anlegefläche ausgeformt ist, die in einem vorgegebenen Einstechwinkel gegen über einer Längserstreckung der Implantiervorrichtung abgeschrägt ist. Durch eine derartige Anlegefläche kann neben der geeigneten Einstichtiefe zusätzlich auch ein bevorzugter Einstechwinkel gegenüber der Haut des Patienten in besonders einfacher Weise eingehalten werden.

Zudem ist am Gehäuse vorteilhafterweise ein Sichtbereich vorgesehen, der beispielsweise durch eine Ausnehmung, eine Einbuchtung oder einen transparenten Abschnitt beziehungsweise ein Fenster des Gehäuses gebildet sein kann, und durch den hindurch ein aus dem Gehäuseaustritt herausragender Abschnitt der Kanüle beziehungsweise eine auf der Kanüle angebrachte Längenabschnittsmarkierung eingesehen werden kann.

In einer bevorzugten Ausführungsform der Implantiervorrichtung sind zudem die Teile, die während eines Setzvorganges mit Gewebe des Patienten in Berührung kommen und dadurch verunreinigt werden, zumindest teilweise austauschbar, um eine Wiederverwendung der restlichen Implantiervorrichtung bei minimiertem Reinigungsbedarf zu ermöglichen. Hierzu ist die Kanüle, beziehungsweise die Kanüle zusammen mit dem Stößel austauschbar an der Implantiervorrichtung gehalten. Darüber hinaus kann hierzu auch der Kanülenhalter oder auch das gesamte zweite Gehäuseteil mit der Kanüle wenigstens teilweise als Einwegeinrichtung ausgebildet sein, die austauschbar am ersten Gehäuseteil festgelegt wird.

In jedem Fall ist es dabei vorteilhaft, wenn die austauschbaren Elemente über eine Sicherungskopplung an der übrigen Vorrichtung festgelegt werden können, und diese Sicherungskopplung erst bei Erreichen der letzten Einrückstellung gelöst werden kann. Auf diese Weise kann verhindert werden, dass die austauschbaren Teile bereits vor Abschluss der jeweiligen Setzvorgänge versehentlich gelöst werden.

Ferner ist es günstig, wenn der mechanische Kraftspeicher zur Wiederverwendung der Implantiervorrichtung mit einem neuen austauschbaren Element durch manuelle Relativbewegung eines Gehäuseabschnittes vorspannbar ist, wobei eine Sicherungshandhabe vorgesehen ist, die in einer Ausgangsstellung die Relativbewegung des Gehäuseabschnittes blockiert und in eine Freigabestellung verlagerbar ist, in der die Relativbewegung freigegeben ist. Hierdurch ist es möglich, die Implantiervorrichtung derart auszuführen, dass deren mechanischer Kraftspeicher jederzeit wieder neu geladen werden kann, wobei ein versehentliches Aufladen der Implantiervorrichtung und eine hieraus resultierende Verletzungsgefahr vermieden wird.

Vorteilhafterweise ist in der Kanüle das wenigstens eine zu implantierende Objekt bereits aufgenommen, so dass die Implantiervorrichtung betriebsbereit zur Verfügung steht. Dabei ist es günstig, wenn das aufgenommene Objekt an der distalen Spitze der Kanüle angeordnet ist, wodurch das Implantat selbst zum Verschließen der Spitze genutzt wird. Dadurch kann ein ungewollter Eintritt von Gewebe in die Kanüle verhindert oder zumindest reduziert werden.

Ferner wird die oben genannte Aufgabe durch eine Implantieranordnung mit einer Implantiervorrichtung in einer der oben genannten Ausführungsformen gelöst, wobei die Implantieranordnung ein separates, zu implantierendes Objekt aufweist das in die Kanüle eingeführt werden kann. Hierdurch steht die Implantiervorrichtung innerhalb eines teilweise wiederverwendbaren aufeinander abgestimmten Sets zur Verfügung.

Dabei ist es günstig, wenn eine Einführhilfe vorgesehen ist, die zur Einführung des wenigstens einen Objekts in den Hohlraum vorgesehen ist und die an einem Ende der Kanüle angebracht werden kann. Eine derartige Einführhilfe, die beispielsweise trichterförmig ausgebildet sein kann, ermöglicht dabei eine vereinfachte Einführung von Implantaten auch in relativ dünne Kanülen.

Darüber hinaus ist es von Vorteil, wenn das wenigstens eine Objekt in einer Hülse aufgenommen ist, die zusammen mit dem Objekt in die Kanüle eingeführt wird. Eine derartige Hülse ermöglicht dabei insbesondere eine besonders komfortable Einführung von zwei oder mehr Implantaten in den Hohlraum der Kanüle.

In den Figuren ist eine beispielhafte Ausführungsform der Erfindung dargestellt. Es zeigen:
- Figur 1: eine perspektivische Ansicht einer erfindungsgemäßen Implantiervor-richtung in einer ersten Einstechstellung,
- Figur 2: einen Längsschnitt durch die Implantiervorrichtung nach Figur 1,
- Figur 2A: eine perspektivische Ansicht einer Anordnung von Festlegemitteln und Festlegegegenmitteln der Implantiervorrichtung nach Figur 1,
- Figur 3: einen Längsschnitt durch die Implantiervorrichtung nach Figur 1 in einer ersten Einrückstellung,
- Figur 4: einen Längsschnitt durch die Implantiervorrichtung nach Figur 1 in einer zweiten Einstechstellung,
- Figur 5: einen Längsschnitt durch die Implantiervorrichtung nach Figur 1 in einer zweiten Einrückstellung,
- Figur 6: einen Längsschnitt durch die Implantiervorrichtung nach Figur 1 in der zweiten Einrückstellung und zusätzlich verlagertem Stößel,
- Figur 7: einen Längsschnitt durch die Implantiervorrichtung nach Figur 1 nach Abschluss aller Setzvorgänge und verschlossenem Gehäuse,
- Figur 8: eine Ansicht der Implantiervorrichtung mit einer alternativen Ausführungsform des Gehäuses,
- Figur 9: eine perspektivische Ansicht der Implantiervorrichtung nach Figur 8 beim Einstechen in eine Hautschicht,
- Figur 10: eine Ansicht der Implantiervorrichtung nach Figur 8 bei Anlage eines Einstechtiefenanschlages an der Hautschicht,
- Figur 11: eine Ansicht der Implantiervorrichtung nach Figur 8 bei Anlage einer Einstichstelle der Haut an einem zweiten Gehäuseteil,
- Figur 12: eine Ansicht einer erfindungsgemäßen Implantiervorrichtung in der ersten Einstechstellung und Einstechen in die Hautschicht,
- Figur 13: eine Ansicht der Implantiervorrichtung nach Figur 12 beim Anliegen des zweiten Gehäuseteils an einer Oberfläche der Hautschicht,
- Figur 14: eine Ansicht der Implantiervorrichtung nach Figur 12 nach einem ersten Setzvorgang,
- Figur 15: eine Ansicht der Implantiervorrichtung nach Figur 12 nach dem Verschwenken um einen Horizontalwinkel,
- Figur 16: eine Ansicht der Implantiervorrichtung nach Figur 12 in einer zweiten Einstechstellung,
- Figur 17: eine Ansicht der Implantiervorrichtung nach Figur 12 nach einem zweiten Setzvorgang,
- Figur 18: eine perspektivische Ansicht der Implantiervorrichtung mit einer weiteren alternativen Ausführungsform des Gehäuses bei abgenommenem zweiten Gehäuseteil,
- Figur 19: einen Längsschnitt durch einen ersten Gehäuseteil der Implantiervorrichtung nach Figur 18,
- Figur 20: einen Längsschnitt durch eine zweiten Gehäuseteil der Implantiervorrichtung nach Figur 18,
- Figur 21: eine perspektivische Ansicht einer alternativen Ausführungsform der Implantiervorrichtung,
- Figur 22: einen Längsschnitt durch die Implantiervorrichtung nach Figur 21 in der ersten Einstechstellung,
- Figur 23: einen Längsschnitt durch die Implantiervorrichtung nach Figur 21 in der ersten Einrückstellung,
- Figur 24: einen Längsschnitt durch die Implantiervorrichtung nach Figur 21 in der zweiten Einstechstellung,
- Figur 25: einen Längsschnitt durch die Implantiervorrichtung nach Figur 21 vor dem zweiten Setzvorgang,
- Figur 26: einen Längsschnitt durch die Implantiervorrichtung nach Figur 21 in der zweiten Einrückstellung,
- Figur 27: einen Längsschnitt durch Gehäusearretiermittel der Implantiervorrichtung in der ersten Einrückstellung des Kanülenhalters,
- Figur 28: eine perspektivische Ansicht einer Kanüle der erfindungsgemäßen Implantiervorrichtung mit angebrachter Einführhilfe,
- Figur 29: eine perspektivische Explosionsansicht der Kanüle mit einer darin einführbaren Hülse und zwei zu implantierenden Objekten,
- Figur 30: eine perspektivische Ansicht der Kanüle mit einem von einem Kreisprofil abweichenden Profil und
- Figur 31: eine vergrößerte Ansicht einer distalen Spitze der Kanüle nach Figur 30.

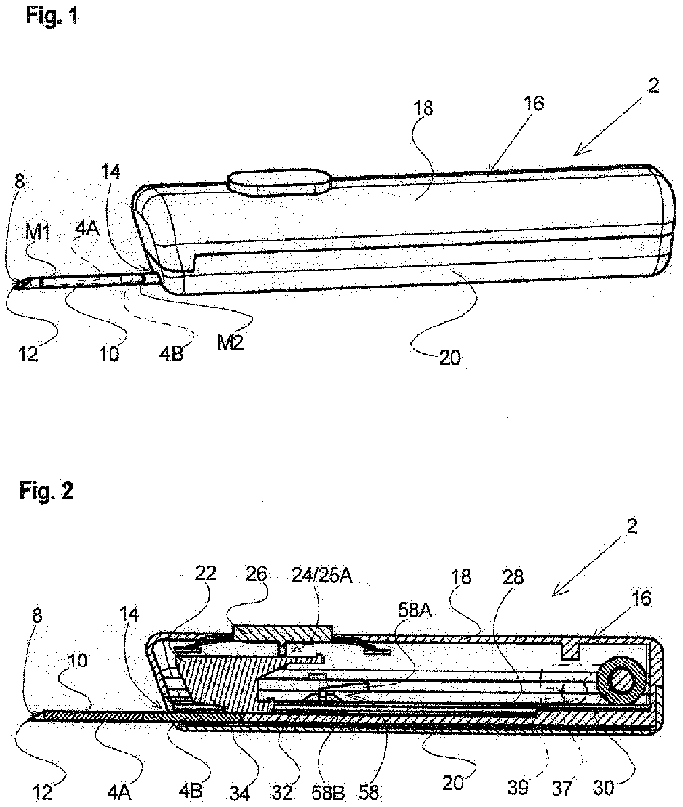

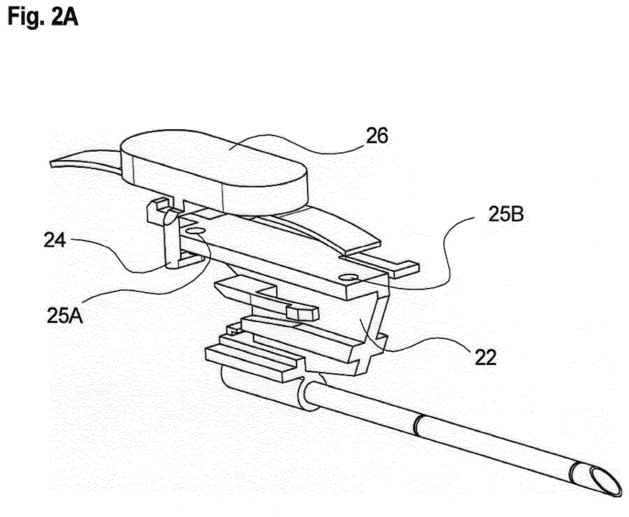

Die Figuren 1 und 2 zeigen eine Implantiervorrichtung 2 zum Setzen von zwei Objekten 4A, 4B in beispielhafter Form von länglichen Hormonimplantaten, die zur Langzeitabgabe von Wirkstoffen für die Empfängnisverhütung einer Patientin implantiert werden können. Alternativ hierzu können die Objekte 4A, 4B auch durch Implantate zur Abgabe sonstiger Medikamente für Menschen oder Tiere gebildet sein oder elektronische Elemente enthalten, die beispielsweise eine Identifikation der jeweiligen Person zur Zutrittskontrolle oder für Bezahldienste ermöglichen oder die als Solarzellen als Teil eines medizinischen oder eines sonstigen elektronischen Implantats dienen.

In jedem Fall sind die zu implantierenden Objekte 4A, 4B für die Durchführung des Setzvorganges in einem Hohlraum 8 einer Kanüle 10 der Implantiervorrichtung 2 aufgenommen. Hierbei ist das erste zu setzende Objekt 4A derart an einer distalen Spitze 12 der Kanüle 10 positioniert, dass es deren Öffnung weitestgehend verschließt.

In der in den Figuren 1 und 2 dargestellten Position befindet sich die Kanüle 10 in einer ersten Einstechstellung, in der sie aus einem Gehäuseaustritt 14 eines Gehäuses 16 der Implantiervorrichtung 2 herausragt. Die Kanüle 10 weist dabei eine erste Längenabschnittsmarkierung M1 und eine zweite Längenabschnittsmarkierung M2 auf, die in der ersten Einstechstellung beide außerhalb des Gehäuses 16 angeordnet sind.

Das Gehäuse 16 der Implantiervorrichtung 2 weist hierbei ein erstes Gehäuseteil 18 auf, dass verschiebbar an einem zweiten Gehäuseteil 20 gehalten ist, das den Gehäuseaustritt 14 bildet.

Wie aus Figur 2 zu entnehmen ist, ist die Kanüle 10 an einem Kanülenhalter 22 befestigt, der innerhalb des Gehäuses 16 geführt verlagert werden kann. In der dargestellten ersten Einstechstellung ist der Kanülenhalter 22 dabei durch Festlegemittel 24 eines Auslöseknopfes 26 gehalten, die mit ersten Festlegegegenmitteln 25A des Kanülenhalters 22 zusammen wirken. Die ineinander greifenden Festlegemittel 24 und Festlegegegenmittel 25a halten dabei den Kanülenhalter 22 entgegen der Vorspannung einer am Kanülenhalter 22 angreifenden Rollfeder 28 eines mechanischen Kraftspeichers 30 in einer distalen Position innerhalb des Gehäuses 16. Der Kraftspeicher 30, die Rollfeder 28 und der Auslöseknopf 26 bilden dabei Rückholmittel zur selbsttätigen Verlagerung des Kanülenhalters 22 in proximaler Richtung.

Ferner weist die Implantiervorrichtung 2 einen Stößel 32 auf der mit dem ersten Gehäuseteil 18 fest verbunden ist und in den Hohlraum 8 der Kanüle 10 hineinragt. Hierbei stützt ein distales Ende 34 des Stößels 32 das zweite zu implantierende Objekt 4B in proximaler Richtung ab.

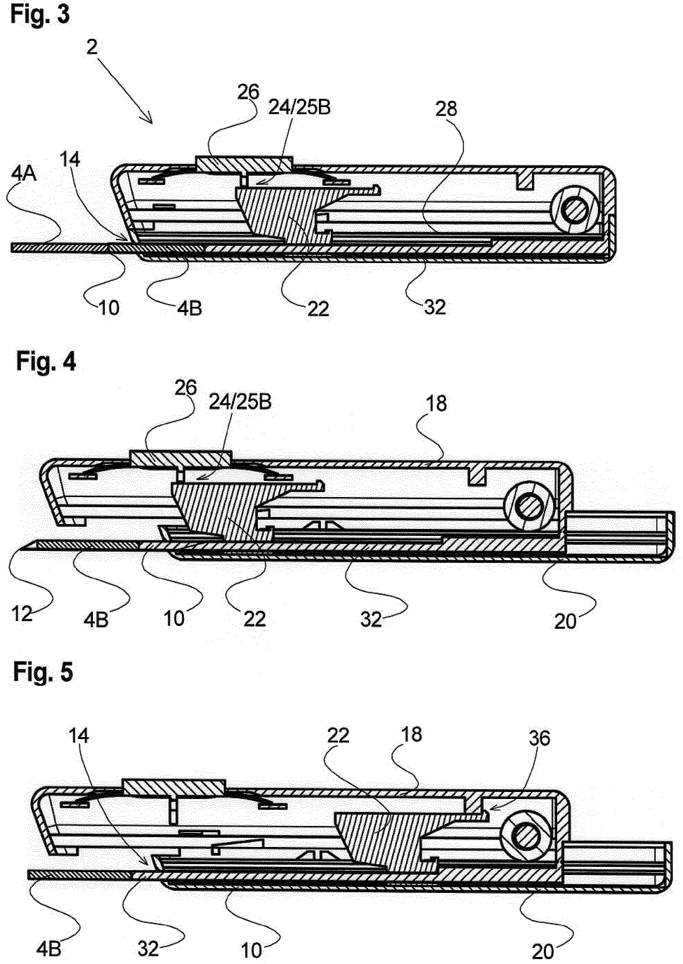

Durch Betätigung des Auslöseknopfes 26 können die Festlegemittel 24 von den ersten Festlegegegenmitteln 25A des Kanülenhalters 22 gelöst werden, wodurch dieser infolge der Vorspannung durch die Rollfeder 28 in proximaler Richtung bis zu einer Anschlagsposition verlagert wird, in der die Festlegemittel 24 des Auslöseknopfes mit zweiten Festlegegegenmitteln 25B des Kanülenhalters 22 in Eingriff kommen, wie sie insbesondere aus Fig. 2A zu entnehmen sind. Gleichzeitig wird hierdurch die Kanüle 10 aus der ersten Einstechstellung über einen ersten Rückhubweg hinweg entlang des Stößels 32 durch den Gehäuseaustritt 14 hindurch in eine erste Einrückstellung gemäß Figur 3 verlagert. Durch diese Bewegung wird, wie aus Figur 3 zu entnehmen ist, das erste Objekt 4A freigesetzt, während das durch den Stößel 32 abgestützte zweite Objekt 4B an die distale Spitze 12 der Kanüle verlagert wird.

Aus dieser ersten Einrückstellung gemäß Figur 3 heraus kann nun das ersten Gehäuseteil 18 durch manuelle Kraftbeaufschlagung gegenüber dem zweiten Gehäuseteil 20 in distaler Richtung verschoben werden. Infolge der Festlegung des Kanülenhalters 22 am Auslöseknopf 26 über die Festlegemittel 24 und Festlegegegenmittel 25B wird dabei auch die Kanüle 10 relativ zum zweiten Gehäuseteil 20 verlagert und gelangt dadurch aus der erste Einrückstellung gemeinsam mit dem Stößel 32 in eine zweite Einstechstellung gemäß Figur 4.

In dieser Stellung gemäß Figur 4 können die Festlegemittel 24 durch nochmalige Betätigung des Auslöseknopfes 26 vollständig von den Festlegegegenmitteln 25B des Kanülenhalters 22 getrennt werden, wodurch dieser infolge der weiteren Vorspannung durch die Rollfeder 28 in proximaler Richtung bis zu einer Endanschlagstellung verlagert wird.

In dieser Endanschlagstellung gemäß Figur 5 bildet der Kanülenhalter 22 zusammen mit dem ersten Gehäuseteil 18 eine Endlagensicherung 36, die beispielsweise durch eine Schnappverbindung geformt ist und die den Kanülenhalter 22 dauerhaft in der Endanschlagstellung sichert. Gleichzeitig wird durch die Verlagerung des Kanülenhalters 22 die Kanüle 10 aus der zweiten Einstechstellung über einen zweiten Rückhubweg hinweg entlang des Stößels 32 und in den Gehäuseaustritt 14 hinein in eine zweite Einrückstellung gemäß Figur 5 verlagert. Durch diese Bewegung wird nun auch das zweite Objekt 4B freigesetzt, während die Kanüle selbst vollständig innerhalb des zweiten Gehäuseteils 20 aufgenommen wird.

Um bei der durch die Rückholmittel generierten selbsttätigen Bewegung des Kanülenhalters 22 in wenigstens eine der Einrückstellungen eine geeignete Geschwindigkeit und eine gleichmäßige Verlagerung zu gewährleisten, kann zudem ein zwischen dem Kraftspeicher 30 und dem Kanülenhalter 22 wirkendes Getriebe 37 und/oder eine Dämpfungseinrichtung 39 vorgesehen sein, wie in Fig. 2 jeweils durch strichpunktierte Linien dargestellt.

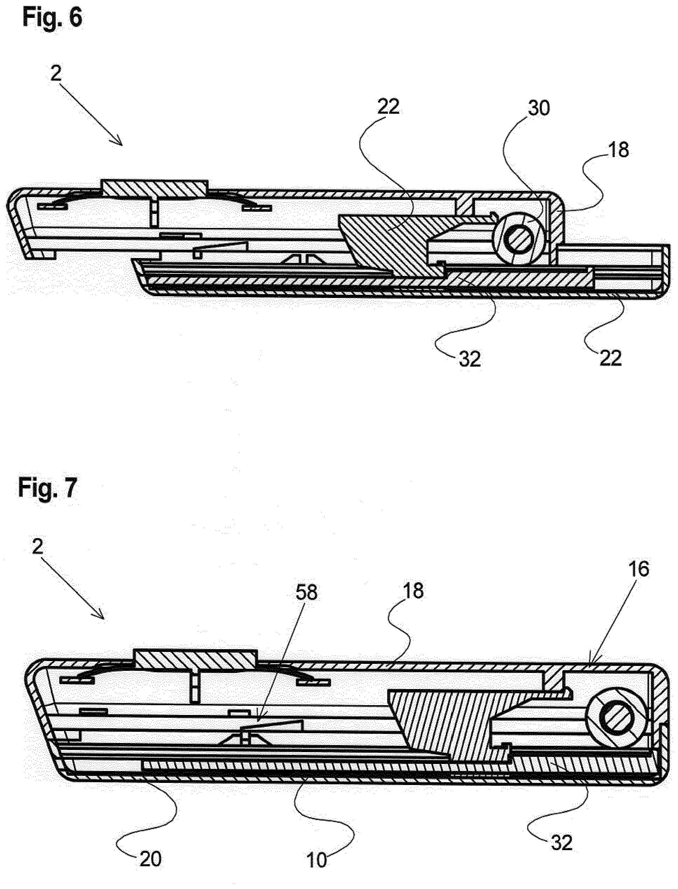

Nach Abschluss des letzten Setzvorganges kann zudem eine weitere Verlagerung des Kanülenhalters 22 in proximaler Richtung in Folge des mechanischen Kraftspeichers 30 vorgesehen werden, durch die der Stößel 32 vom ersten Gehäuseteil 18 abgetrennt und vollständig in den zweiten Gehäuseteil 20 verlagert werden kann, wie in Figur 6 dargestellt.

Anschließend kann dann das ersten Gehäuseteil 18 durch manuelle Kraftaufbringung in proximaler Richtung gegenüber dem zweiten Gehäuseteil 20 verschoben werden, um das Gehäuse 16 mit der darin vollständig aufgenommenen Kanüle 10 und dem vollständig aufgenommenen Stößel 32 gemäß Figur 7 verschließen und gegebenenfalls entsorgen zu können.

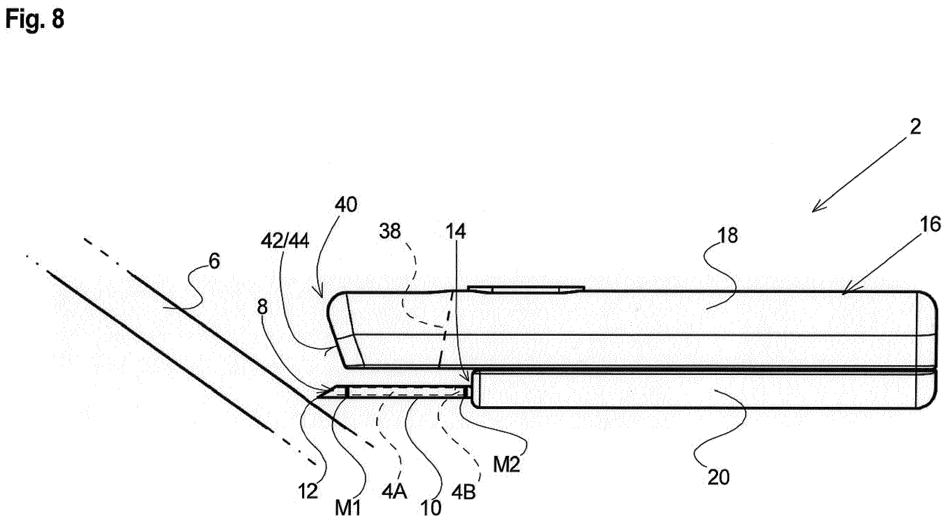

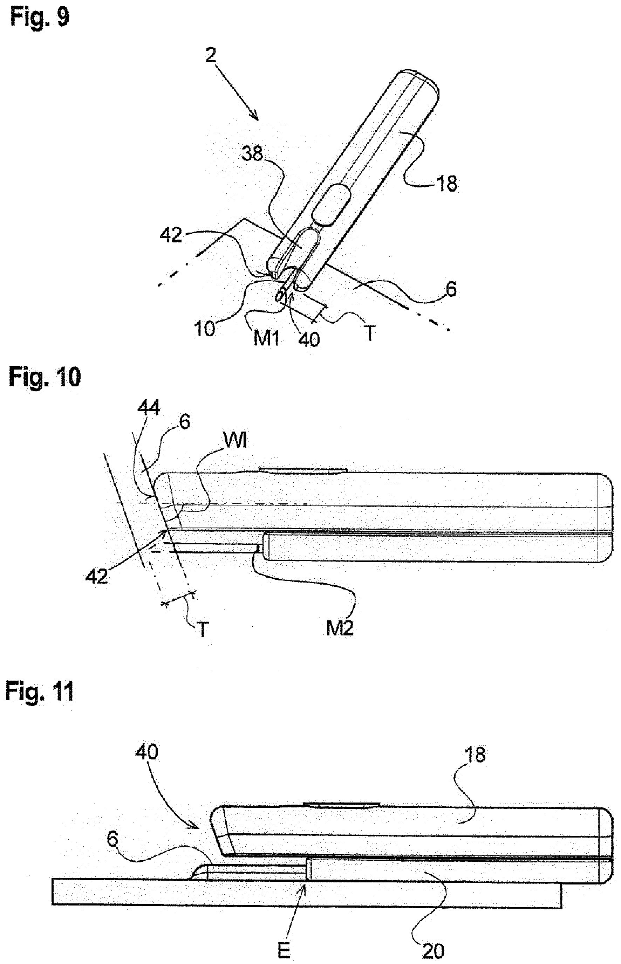

Figur 8 zeigt die Implantiervorrichtung 2 mit einer alternativen Ausführungsform des Gehäuses 16. Dieses weist an einem distalen Ende 40 ein gegenüber dem ersten Gehäuseteil 18 verkürztes zweites Gehäuseteil 20 sowie einen Sichtbereich 38 auf. Dabei bildet das erste Gehäuseteil 18 am distalen Ende 40 einen Anschlag 42 aus, der wie aus den Figuren 9 und 10 zu entnehmen ist, eine Einstichtiefe T der Kanüle 10 begrenzt. Durch den durch eine Einbuchtung gebildeten Sichtbereich 38 wird dabei gleichzeitig eine gute Einsehbarkeit eines Kanülenabschnittes beziehungsweise der Längenabschnittmarkierung M1 ermöglicht.

Wie in Figur 10 dargestellt, ist der Anschlag 42 dabei durch eine Anlegefläche 44 gebildet, die gegenüber einer Längserstreckung der Implantiervorrichtung 2 in einem vorgegebenen Einstechwinkel WI abgeschrägt ist.

Wie aus Figur 11 zu entnehmen ist, kann durch die verkürzte Ausführung des zweiten Gehäuseteils 20 gegenüber dem ersten Gehäuseteil 18 zudem das distale Ende des zweiten Gehäuseteils 20 als zusätzlicher Endanschlag dienen, an dem eine Einstichstelle E zur Anlage kommt, sobald die Kanüle 10 bis zur zweiten Längenabschnittsmarkierung M2 in eine Hautschicht 6 eingeschoben ist, wie in Figur 11 dargestellt.

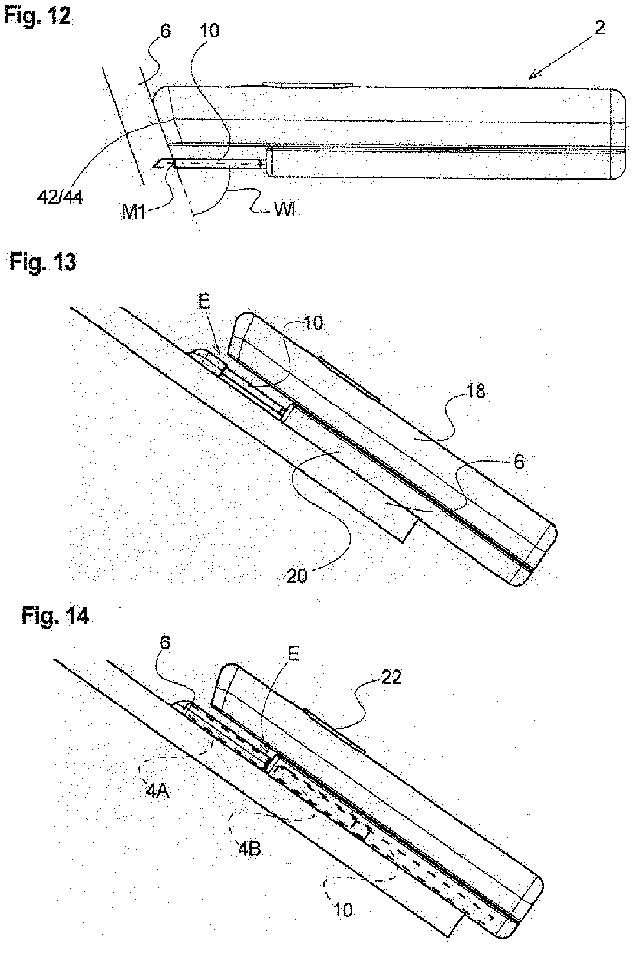

Das Setzen der zu implantierenden Objekte 4A, 4B wird nachfolgend anhand der Ausführungsvariante der Implantiervorrichtung 2 gemäß der Figuren 8 bis 11 beschrieben:
Zum Setzen des ersten Objektes 4A wird dabei, wie aus Figur 12 zu entnehmen ist, die aus dem Gehäuse 16 herausragende Kanüle 10 zunächst bis zur ersten Längenabschnittsmarkierung M1 in die Hautschicht 6 der Patientin eingestochen, wobei die Anlegefläche 44 als Anschlag 42 zur Begrenzung der Einstichtiefe fungiert. Das Einstechen erfolgt hierbei wie dargestellt unter dem Einstechwinkel WI.

Anschließend wird die Implantiervorrichtung 2 derart um die Einstichstelle E herum geschwenkt, dass das zweite Gehäuseteil 20, gemäß Figur 13, auf der Oberfläche der Hautschicht 6 anliegt. Daraufhin wird die Implantiervorrichtung 2 so weit in Richtung der Einstichstelle E verschoben, bis die Kanüle 10 bis zur zweiten Längenabschnittsmarkierung M2 in die Einstichstelle E hinein verlagert ist beziehungsweise bis die Einstichstelle E am distalen Ende des zweiten Gehäuseteils 20 anliegt.

Durch Betätigung des Auslöseknopfes 22 wird die Kanüle 10 über den ersten Rückhubweg hinweg in den zweiten Gehäuseteil 20 hinein in die erste Einrückstellung gemäß Figur 14 verlagert. Das erste Objekt 4A verbleibt hierbei, wie dargestellt, an der Einstichstelle E unter der Hautschicht 6.

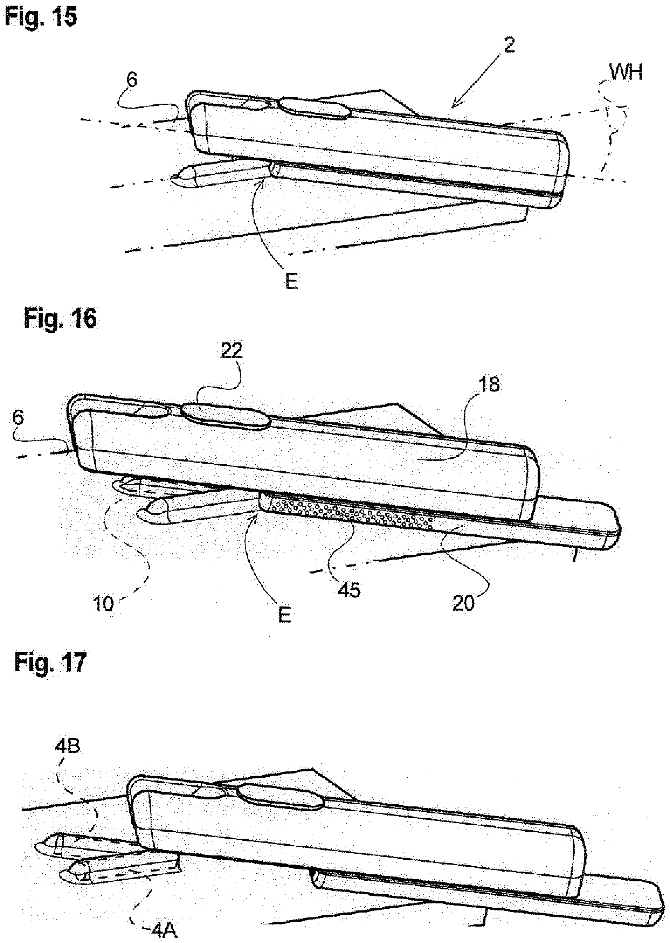

Zum anschließenden Setzen des zweiten Objektes 4B wird die Implantiervorrichtung 2 an der Oberfläche der Hautschicht 6 um die Einstichstelle E herum über einen Horizontalwinkel WH verdreht, wie in Figur 15 dargestellt.

Anschließend wird der erste Gehäuseteil 18 durch manuelle Kraftbeaufschlagung relativ zum zweiten Gehäuseteil 20 in Richtung der Einstichstelle E verschoben. Um hierbei eine stabile Position des zweiten Gehäuseteils 20 gewährleisten zu können, sind an dessen Oberfläche rutschhemmende Mittel 45 vorgesehen, die beispielsweise durch eine Gummierung oder durch Noppen gebildet sein können.

Durch die Relativbewegung zwischen beiden Gehäuseteilen 18, 20 gelangt die Kanüle 10 aus der ersten Einrückstellung in die zweite Einstechstellung gegenüber dem zweiten Gehäuseteil 20, wobei sie unterhalb der Hautschicht 6 verlagert wird bis die Einstichstelle E wiederum am zweiten Gehäuseteil 20 anliegt, wie aus Figur 16 zu entnehmen ist.

Durch nochmaliges Betätigen des Auslöseknopfes 22 wird die Kanüle 10 aus der zweiten Einstechstellung heraus entlang des zweiten Rückhubweges in die zweite Einrückstellung innerhalb des zweiten Gehäuseteils 20 verlagert. Dabei verbleibt das zweite Objekt 4B, wie in Figur 17 dargestellt, in einem dem Horizontalwinkel WH entsprechenden Winkel zum ersten Objekt 4A unter der Hautschicht 6.

Im Anschluss an diesen zweiten beziehungsweise letzten Setzvorgang ist sowohl die Kanüle 10 als auch der Stößel 32, wie oben beschrieben, vollständig im zweiten Gehäuseteils 20 aufgenommen. Durch manuelles Verschieben des ersten Gehäuseteils 18 gegenüber dem zweiten Gehäuseteil 20 kann daraufhin das Gehäuse 16 geschlossen und sicher aufbewahrt beziehungsweise entsorgt werden.

Zur sicheren Entsorgung aller Teile, die während der Setzvorgänge insbesondere durch Gewebe- und sonstige Schmutzpartikel verunreinigt worden sind, kann die gesamte Implantiervorrichtung 2 als Einwegprodukt konzipiert sein, dass als Ganzes entsorgt wird. Alternativ hierzu ist es auch möglich, die Kanüle 10 über eine lösbare Verbindung am Kanülenhalter 22 und/oder den Stößel 32 über eine lösbare Verbindung, wie beispielsweise einer Steckverbindung, am ersten Gehäuseteil 18 zu befestigen, so dass beide Teile nach Abschluss der Setzvorgänge durch neue Teile ersetzt werden können. Zudem ist es alternativ möglich, die Kanüle 10 in Verbindung mit zumindest Teilen des Kanülenhalters 22 als Einwegeinrichtung auszubilden, die austauschbar an der übrigen Implantiervorrichtung 2 festgelegt werden kann.

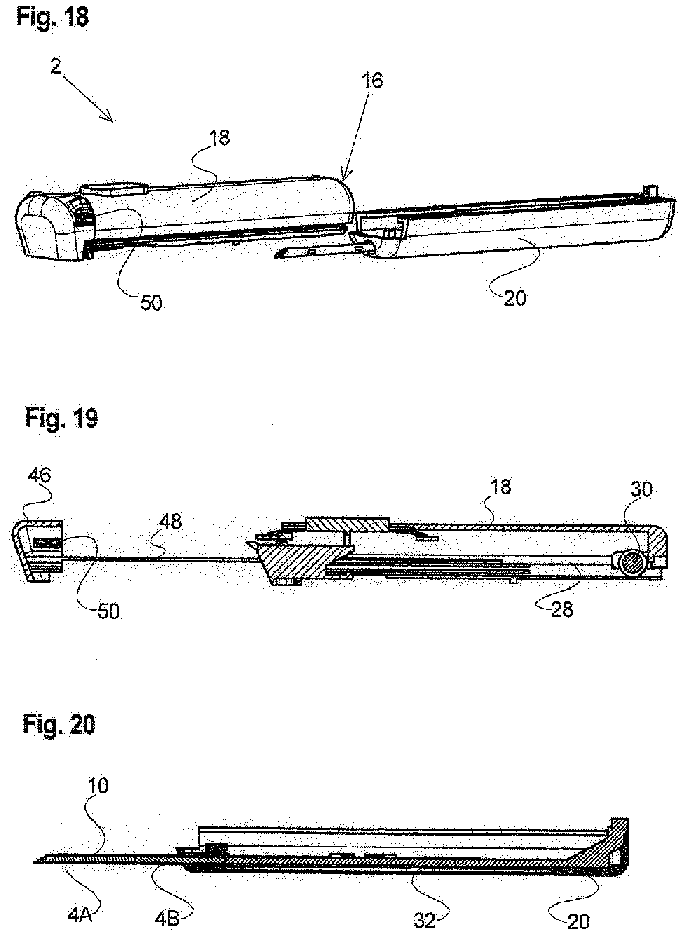

In den Figuren 18 bis 20 ist eine weitere alternative Ausführungsform des Gehäuses 16 der Implantiervorrichtung 2 dargestellt, bei der das gesamte zweite Gehäuseteil 20 als Einwegeinrichtung ausgebildet und austauschbar am ersten Gehäuseteil 18 gehalten ist. Mit dem zweiten Gehäuseteil 20 können dabei auch die Kanüle 10 und der Stößel 32 entsorgt beziehungsweise durch neue Teile ersetzt werden. Um hierbei ein versehentliches Lösen des zweiten Gehäuseteils 20 während des Betriebs zu verhindern, ist zwischen dem ersten Gehäuseteil 18 und dem zweiten Gehäuseteil 20 eine Sicherungskopplung (nicht dargestellt) vorgesehen, die erst in der letzten Einrückstellung der Kanüle 10 beziehungsweise des Kanülenhalters 22 gelöst ist oder gelöst werden kann. Hierdurch kann das zweite Gehäuseteil 20 nach Abschluss aller vorgesehenen Implantiervorgänge von der übrigen Implantiervorrichtung 2 abgenommen werden, wie in Figur 18 dargestellt.

Um die verbleibende Implantiervorrichtung 2 hierbei wiederverwenden zu können, sind am ersten Gehäuseteil 18 Mittel zum erneuten Vorspannen des mechanischen Kraftspeichers 30 beziehungsweise der Rollfeder 28 vorgesehen, die insbesondere aus Figur 19 zu entnehmen sind. Diese umfassen einen abnehmbaren Gehäuseabschnitt 46 des ersten Gehäuseteils 18, der über ein Zugglied 48 mit der Rollfeder 28 verbunden ist. Dieser Gehäuseabschnitt 46 kann durch Drücken einer Sicherungshandhabe 50, die beispielhaft als Druckknopf am Gehäuseabschnitt 46 ausgebildet ist, vom übrigen ersten Gehäuseteil 18 gelöst und weg gezogen werden, um den mechanischen Kraftspeicher 30 über das Zugglied 48 und die Rollfeder 28 neu zu laden, wie in Figur 19 dargestellt.

Hiernach wird der Gehäuseabschnitt 46 wieder mit dem übrigen ersten Gehäuseteil 18 verbunden, so dass anschließend ein neues zweites Gehäuseteil 20 am ersten Gehäuseteil 18 angebracht werden kann. Wie aus Figur 20 zu entnehmen ist, ist an diesem neuen zweiten Gehäuseteil 20 dabei sowohl eine neue Kanüle 10 mit neuen zu implantierenden Objekten 4A, 4B als auch ein neuer Stößel 32 zur positionsstabilen Festlegung am ersten Gehäuseteil 18 vorgesehen.

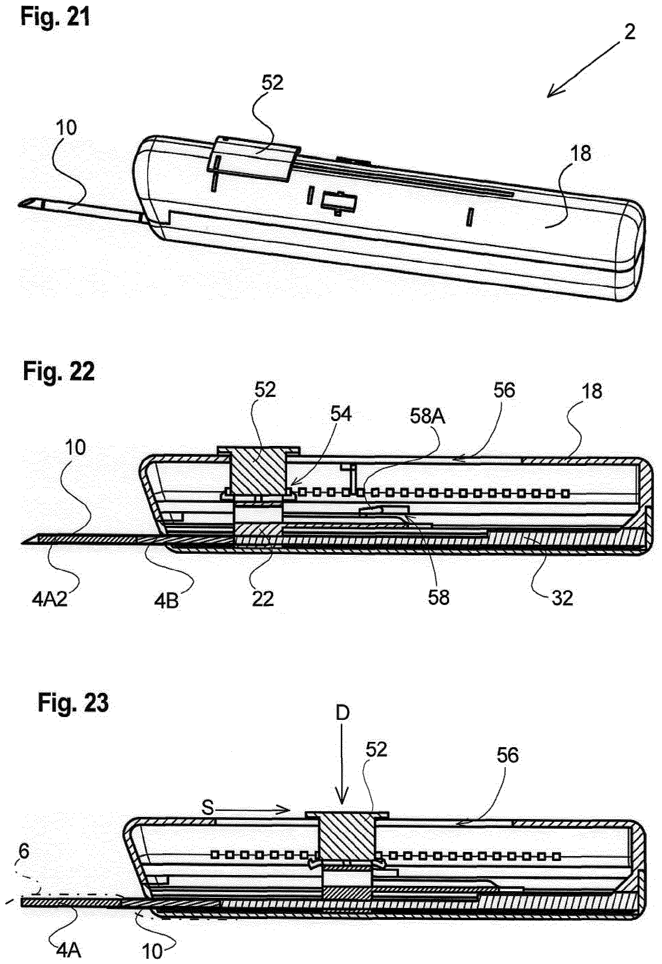

Figur 21 zeigt eine weitere Ausführungsform der Implantiervorrichtung 2, die einen mit den oben beschriebenen Ausführungsformen der Implantiervorrichtung 2 im Wesentlichen übereinstimmenden Funktionsablauf beim implantieren der Objekte 4A und 4B aufweist. Hierbei unterscheidet sich die Ausführungsform nach Figur 21 dadurch, dass die Rückholmittel zur Verlagerung der Kanüle 10 über die Rückhubwege hinweg durch eine manuelle Kraftbeaufschlagung betätigt werden.

Hierzu ist am ersten Gehäuseteil 18 ein Schiebeknopf 52 vorgesehen, der in der ersten Einstechstellung über eine Arretierung 54, die beispielhaft durch Schnapphaken und mit diesen zusammenwirkenden Eingriffselemente gebildet ist, in einer distalen Anschlagsposition einer Führung 56 festgelegt ist. Der Schiebeknopf 52 ist dabei mit dem Kanülenhalter 22 verbunden, an dem entsprechend der oben beschriebenen Ausführungsformen der Implantiervorrichtung 2 die Kanüle 10 gehalten ist, in die der Stößel 32 hineinragt.

Nach dem Einstechen und Verschieben der Kanüle 10 in eine erste Einstichstellung unter der Hautschicht 6 kann die Arretierung des Schiebeknopfes 52 am ersten Gehäuseteil 18 durch manuelle Aufbringung einer Kraft D gelöst und der Schiebeknopf 52 mittels einer manuellen Schiebekraft S entlang der Führung 56 in die erste Einrückstellung gemäß Figur 23 verlagert werden. Hierbei wird, wie dargestellt, das erste Objekt 4A unter der Hautschicht 6 abgelegt.

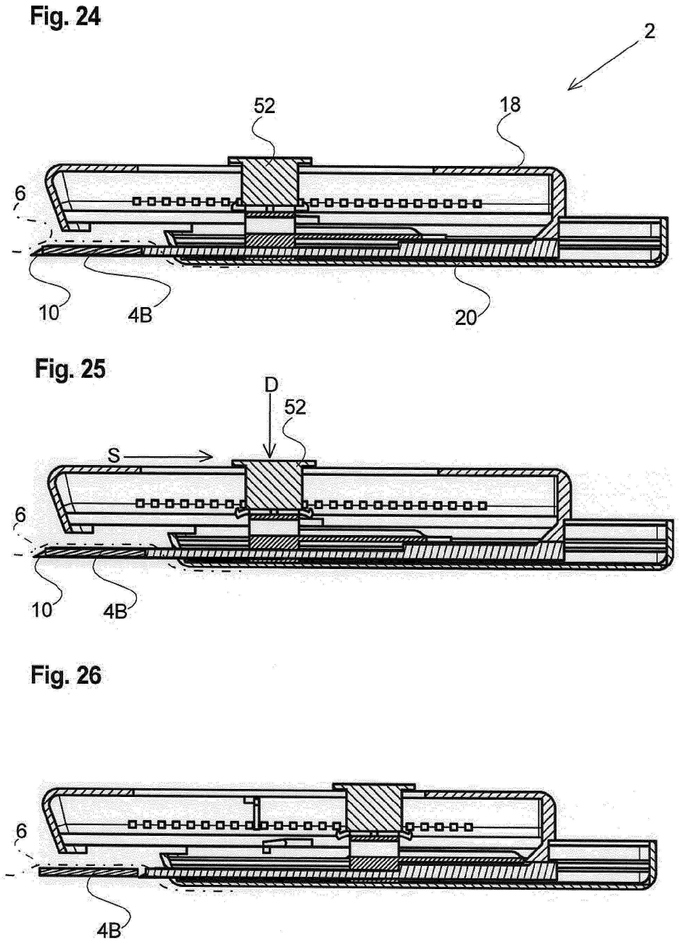

Zur Vorbereitung des zweiten Setzvorganges wird die Beaufschlagung des Schiebeknopfes 52 mit der Kraft D beendet und dadurch dessen Arretierung 54 am ersten Gehäuseteil 18 wieder hergestellt. Anschließend kann das erste Gehäuseteil 18 zusammen mit dem Kanülenhalter 22 und dem Stößel 32 in distaler Richtung gegenüber dem zweiten Gehäuseteil 20 in die zweite Einstechstellung verschoben werden, um die Kanüle 10 im zweiten Implantierbereich unter der Hautschicht 6 zu positionieren, wie in Figur 24 dargestellt.

Zum Setzen des zweiten Objektes 4B wird der Schiebeknopf 52 erneut mit der Kraft D zum Lösen der Arretierung 54 beaufschlagt wie in Figur 25 dargestellt und gleichzeitig durch Beaufschlagung mit der Schiebekraft S in die zweite Einrückstellung gemäß Figur 26 verlagert, wobei das zweite Objekt 4B unter der Hautschicht 6 abgelegt wird.

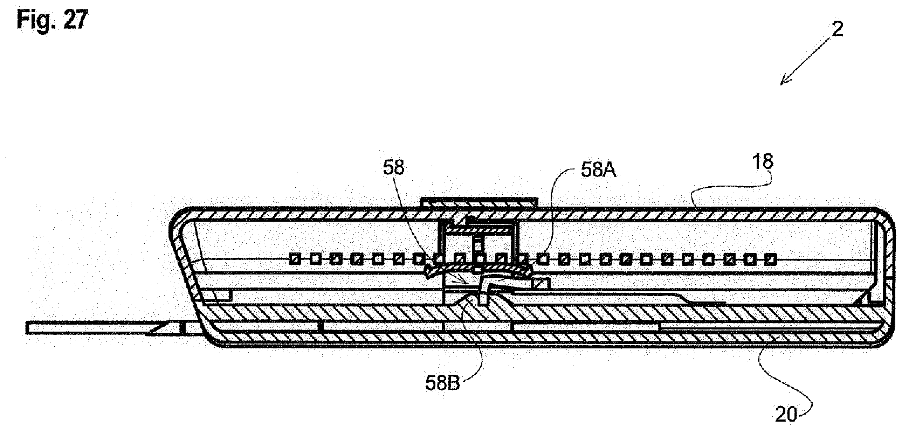

Unabhängig von der jeweiligen Ausführungsform der Implantiervorrichtung 2 können zwischen dem ersten Gehäuseteil 18 und dem zweiten Gehäuseteil 20 Gehäusearretiermittel 58 vorgesehen sein, wie sie in Fig. 27 beispielhaft anhand eines Arretierhakens 58A und einer mit diesem zusammen wirkenden Arretieraufnahme 58B dargestellt sind. Der Arretierhaken 58A steht dabei vor dem Erreichen der ersten Einrückstellung in einer Arretierstellung, in der er in die Arretieraufnahme 58B eingreift und dadurch, das erste Gehäuseteil 18 am zweiten Gehäuseteil 20 festlegt, wie beispielsweise aus Fig. 2 und 22 zu entnehmen ist. Durch diese gegenseitige Festlegung kann bis zum Erreichen der ersten Einrückstellung des Kanülenhalters 22 eine störende Relativbewegung während des Einstechens der Kanüle 10 verhindert werden. Sobald jedoch die erste Einrückstellung erreicht ist, gelangt der Arretierhaken 58A außer Eingriff mit der Arretieraufnahme 58B, so dass nun eine Relativbewegung zwischen den Gehäuseteilen 18, 20 möglich ist. Zudem kann vorgesehen sein, dass die Arretiermittel 58 nach dem Abschluss des letzten Implantiervorganges beim Verschließen des Gehäuses gemäß. Fig. 7 wieder in die Arretierstellung kommen, um in dieser Endstellung eine Relativbewegung zwiswchen den Gehäuseteilen 18, 20 erneut zu blockieren.

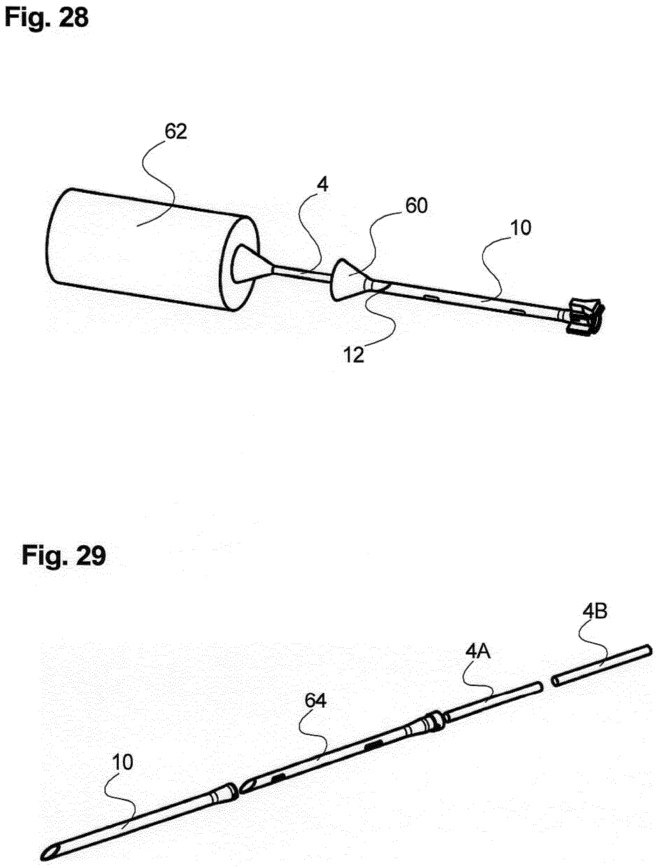

Wie vorangehend dargestellt, können die wenigstens zwei zu implantierenden Objekte 4A, 4B vor Verwendung der Implantiervorrichtung 2 bereits anwendungsfertig in der Kanüle 10 aufgenommen sein. Alternativ hierzu kann die Implantiervorrichtung 2 auch als Teil einer Implantieranordnung konzipiert sein, bei der wenigstens ein zu implantierendes Objekt 4 zunächst als separates Element vorliegt, das vor Verwendung der Implantiervorrichtung in die Kanüle 10 eingeführt werden muss. Hierzu kann eine Einführhilfe 60 vorgesehen werden, die wie in Figur 28 dargestellt, beispielsweise einen trichterförmigen Teil aufweist, der an der distalen Spitze 12 der Kanüle 10 angebracht werden kann, um das betreffende Objekt 4 mittels einer Handhabe 62 leichter in den Hohlraum 8 der Kanüle 10 einführen zu können.

Um insbesondere auch zwei oder mehrere Objekte 4A, 4B leichter in die Kanüle 10 einführen zu können, können diese alternativ oder zusätzlich in einer Hülse aufgenommen werden, wie sie aus Figur 29 zu entnehmen ist.

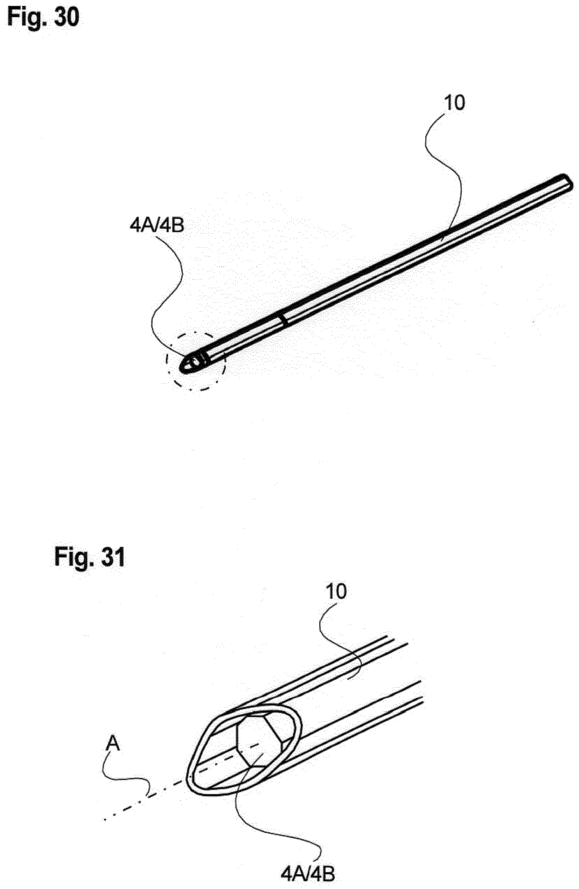

Unabhängig von der jeweiligen Ausführungsform kann die Implantiervorrichtung 2 zum Implantieren von Objekten 4A, 4B mit unterschiedlicher Funktion verwendet werden. Insbesondere im Falle von Objekten 4A, 4B, bei denen die Funktionsweise von einer geeigneten Drehposition bezüglich ihrer Längsachse A gegenüber der Hautschicht 6 abhängt, kann die Kanüle 10 beziehungsweise deren Hohlraum 8, wie aus den Figuren 30 und 31 zu entnehmen ist, einen von einer Kreisform abweichenden Querschnitt aufweisen, durch den das entsprechend geformte Objekt 4A, 4B bezüglich der Längsachse A lagestabil geführt und gesetzt werden kann. Hierzu kann der Querschnitt beispielsweise mehreckig, oval, nierenförmig, elliptisch oder ähnlich ausgeführt sein.

## Patentansprüche

1. Implantiervorrichtung (2) für die Positionierung mindestens eines Objektes (4A; 4B) unterhalb der Haut eines Patienten
mit einem Gehäuse (16),
einem gegenüber dem Gehäuse (16) verlagerbaren Kanülenhalter (22), an dem eine Kanüle (10) mit einer distalen Spitze (12) und einem Hohlraum (8) für die Aufnahme des mindestens einen zu implantierenden Objektes (4A; 4B) anbringbar ist,
und einem gegenüber dem Gehäuse (16) festlegbaren Stößel (32), der ein im Hohlraum (8) der Kanüle (10) positionierbares distales Ende (34) aufweist,
wobei die Kanüle (10) von einer ersten Einstechstellung gegenüber einem Gehäuseaustritt (14) über einen ersten Rückhubweg entlang des Stößels (32) in eine erste Einrückstellung verlagerbar ist, um ein im Hohlraum (8) aufgenommenes erstes Objekt (4A) abzulegen,
und die Kanüle (10) von der ersten Einrückstellung gemeinsam mit dem Stößel (32) in eine zweite Einstechstellung gegenüber dem Gehäuseaustritt (14) und
von der zweiten Einstechstellung über einen zweiten Rückhubweg entlang des Stößels (32) in eine zweite Einrückstellung verlagerbar ist, um ein im Hohlraum (8) aufgenommenes zweites Objekt (4B) abzulegen,
**dadurch gekennzeichnet, dass** der Kanülenhalter (22) zur Verlagerung in die zweite Einstechstellung an einem ersten Gehäuseteil (18) festlegbar ist, das mit dem distalen Ende (34) des Stößels (32) fest verbunden ist und an einem zweiten, den Gehäuseaustritt (14) bildenden Gehäuseteil (20) verlagerbar gehalten ist.

2. Implantiervorrichtung nach Anspruch 1, **dadurch gekennzeichnet, dass** das erste Gehäuseteil (18) verschiebbar am zweiten Gehäuseteil (20) gelagert ist.

3. Implantiervorrichtung nach Anspruch 1 oder 2, **dadurch gekennzeichnet, dass** das zweite Gehäuseteil (20) an einer Außenseite rutschhemmende Mittel (45) aufweist.

4. Implantiervorrichtung nach einem der Ansprüche 1 bis 3, **dadurch gekennzeichnet, dass** zwischen dem ersten Gehäuseteil (18) und dem zweiten Gehäuseteil (20) Gehäusearretiermittel (58) vorgesehen sind, die bis zum Erreichen der ersten Einrückstellung der Kanüle (10) beide Gehäuseteile (18, 20) aneinander festlegen.

5. Implantiervorrichtung nach einem der Ansprüche 1 bis 4, **dadurch gekennzeichnet, dass** Rückholmittel vorgesehen sind, mittels denen die Verlagerung der Kanüle (10) über wenigstens einen der Rückhubwege hinweg selbsttätig ausführbar ist, wobei die Rückholmittel einen mechanischen Kraftspeicher (30) aufweisen, mittels dem der Kanülenhalter (22) verlagerbar ist.

6. Implantiervorrichtung nach Anspruch 5, **dadurch gekennzeichnet, dass** die Rückholmittel ein Getriebe (37) aufweisen, über das der Kanülenhalter (22) verlagerbar ist.

7. Implantiervorrichtung nach Anspruch 5 oder 6, **dadurch gekennzeichnet, dass** die Rückholmittel eine Dämpfungseinrichtung (39) aufweisen, mittels der eine Verlagerungsgeschwindigkeit während der Verlagerung über wenigstens einen der Rückhubwege hinweg einstellbar ist.

8. Implantiervorrichtung nach einem der Ansprüche 5 bis 7, **dadurch gekennzeichnet, dass** die Rückholmittel einen Auslöseknopf (26) aufweisen, mittels dem die Verlagerung über wenigstens einen der Rückhubwege auslösbar ist.

9. Implantiervorrichtung nach Anspruch 8, **dadurch gekennzeichnet, dass** an dem Auslöseknopf (26) Festlegemittel (24) vorgesehen sind, die mit Festlegegegenmitteln (25) des Kanülenhalters (22) lösbar verbindbar sind.

10. Implantiervorrichtung nach einem der Ansprüche 1 bis 9, **dadurch gekennzeichnet, dass** die Verlagerung über wenigstens einen der Rückhubwege manuell ausführbar ist.

11. Implantiervorrichtung nach Anspruch 10, **dadurch gekennzeichnet, dass** zur manuellen Verlagerung ein am Gehäuse (16) verschiebbarer Schiebeknopf (52) vorgesehen ist, der durch Kraftbeaufschlagung aus einer Arretierung lösbar ist.

12. Implantiervorrichtung nach einem der Ansprüche 1 bis 11, **dadurch gekennzeichnet, dass** die Kanüle (10) in einer letzten Einrückstellung vollständig innerhalb des Gehäuses (16) aufgenommen ist, wobei eine Endlagensicherung (36) vorgesehen ist, mittels der der Kanülenhalter (22) in der letzten Einrückstellung gegenüber dem Gehäuse (16) festlegbar ist.

13. Implantiervorrichtung nach Anspruch 12, **dadurch gekennzeichnet, dass** der Stößel (32) in der zweiten Einrückstellung vom Gehäuse (16) trennbar ist.

14. Implantiervorrichtung nach einem der Ansprüche 1 bis 13, **dadurch gekennzeichnet, dass** die Kanüle (10) und der Stößel (32) einen von einem Kreisprofil abweichenden Querschnitt aufweisen.

15. Implantiervorrichtung nach einem der Ansprüche 1 bis 14, **dadurch gekennzeichnet, dass** an einem distalen Ende (40) des Gehäuses (16) ein Anschlag (42) zur Einstechtiefenbegrenzung vorgesehen ist.

16. Implantiervorrichtung nach Anspruch 15, **dadurch gekennzeichnet, dass** an dem Anschlag (42) eine in einem vorgegebenen Einstechwinkel (WI) abgeschrägte Anlegefläche (44) ausgebildet ist.

17. Implantiervorrichtung nach einem der Ansprüche 1 bis 16, **dadurch gekennzeichnet, dass** am Gehäuse (16) ein Sichtbereich (38) vorgesehen ist, durch den hindurch ein aus dem Gehäuseaustritt (14) heraus stehender Abschnitt der Kanüle (10) eingesehen werden kann.

18. Implantiervorrichtung nach einem der Ansprüche 1 bis 17, **dadurch gekennzeichnet, dass** der Stößel (32) zusammen mit der Kanüle (10) austauschbar gehalten ist.

19. Implantiervorrichtung nach Anspruch 18, **dadurch gekennzeichnet, dass** der Kanülenhalter (22) wenigstens teilweise als Einwegeinrichtung ausgebildet ist, die austauschbar an der übrigen Vorrichtung festlegbar ist.

20. Implantiervorrichtung nach Anspruch 18, **dadurch gekennzeichnet, dass** das den Gehäuseaustritt (14) bildende zweite Gehäuseteil (20) mit der Kanüle (10) austauschbar am ersten Gehäuseteil (18) gehalten ist.

21. Implantiervorrichtung nach einem der Ansprüche 18 bis 20, **dadurch gekennzeichnet, dass** die austauschbaren Elemente über eine Sicherungskopplung an der übrigen Vorrichtung festlegbar sind, wobei die Sicherungskopplung in der letzten Einrückstellung lösbar ist.

22. Implantiervorrichtung nach einem der Ansprüche 18 bis 21, **dadurch gekennzeichnet, dass** der mechanische Kraftspeicher (30) zur Wiederverwendung der Implantiervorrichtung (2) mit einem neuen austauschbaren Element durch manuelle Relativbewegung eines Gehäuseabschnittes (46) vorspannbar ist, wobei eine Sicherungshandhabe (50) vorgesehen ist, die in einer Ausgangsstellung die Relativbewegung des Gehäuseabschnittes (46) blockiert und in eine Freigabestellung verlagerbar ist, in der die Relativbewegung freigegeben ist.

23. Implantiervorrichtung nach einem der Ansprüche 18 bis 22, **dadurch gekennzeichnet, dass** in der Kanüle (10) wenigstens ein zu implantierendes Objekt (4A; 4B) aufgenommen ist, das an der distalen Spitze (12) der Kanüle (10) angeordnet ist und ferner wenigstens ein separates, zu implantierendes Objekt (4A; 4B) vorgesehen ist, das in die Kanüle (10) einführbar ist.

24. Implantiervorrichtung nach Anspruch 23, **dadurch gekennzeichnet, dass** eine Einführhilfe (60) zur Einführung des wenigstens einen Objekts (4A; 4B) in den Hohlraum (8) vorgesehen ist, die an einem Ende der Kanüle (10) anbringbar ist.

25. Im plantiervorrichtung nach Anspruch 23 oder 24, **dadurch gekennzeichnet, dass** das wenigstens eine Objekt (4A; 4B) in einer Hülse (64) aufgenommen ist, die in die Kanüle (10) einführbar ist.

## Claims

1. An implantation device (2) for positioning at least one object (4A; 4B) under the skin of a patient,
comprising a housing (16),
a cannula holder (22) displaceable with respect to the housing (16), to which a cannula (10) with a distal tip (12) and a cavity (8) can be attached for receiving the at least one object (4A; 4B) to be implanted,
and a tappet (32) which can be fixed with respect to the housing (16), which has a distal end (34) that can be positioned in the cavity (8) of the cannula (10),
wherein the cannula (10) is displaceable from a first insertion position with respect to a housing outlet (14) to a first operating position via a first return stroke path along the tappet (32), in order to deposit a first object (4A) received in the cavity (8),
and the cannula (10) is displaceable from the first operating position together with the tappet (32) to a second insertion position with respect to the housing outlet (14) and
from the second insertion position to a second operating position via a second return stroke path along the tappet (32), in order to deposit a second object (4B) received in the cavity (8),
**characterized in that** the cannula holder (22) can be fixed for displacement to the second insertion position on a first housing part (18), which is rigidly connected to the distal end (34) of the tappet (32) and is displaceably held on a second housing part (20) forming the housing outlet (14).

2. The implantation device according to claim 1, **characterized in that** the first housing part (18) is slidingly mounted on the second housing part (20).

3. The implantation device according to claim 1 or 2, **characterized in that** the second housing part (20) has slipinhibiting means (45) on an exterior.

4. The implantation device according to any of claims 1 to 3, **characterized in that** housing locking means (58) are provided between the first housing part (18) and the second housing part (20), which fix the two housing parts (18, 20) to each other until reaching the first insertion position of the cannula (10).

5. The implantation device according to any of claims 1 to 4, **characterized in that** retrieval means are provided, by means of which the displacement of the cannula (10) can be automatically executed over at least one of the return stroke paths, wherein the retrieval means have a mechanical energy store (30), by means of which the cannula holder (22) is displaceable.

6. The implantation device according to claim 5, **characterized in that** the retrieval means have a gear mechanism (37), via which the cannula holder (22) is displaceable.

7. The implantation device according to claim 5 or 6, **characterized in that** the retrieval means have a damping device (39), by means of which a displacement speed is adjustable during the displacement over at least one of the return stroke paths.

8. The implantation device according to any of claims 5 to 7, **characterized in that** the retrieval means have a trigger button (26), by means of which the displacement can be triggered over at least one of the return stroke paths.

9. The implantation device according to claim 8, **characterized in that** fixing means (24) are provided on the trigger button (26), which can be detachably connected to counter fixing means (25) of the cannula holder (22).

10. The implantation device according to any of claims 1 to 9, **characterized in that** the displacement can be manually executed over at least one of the return stroke paths.

11. The implantation device according to claim 10, **characterized in that** a sliding button (52) that is slidable on the housing (16) is provided for manual displacement, said sliding button being detachable from a lock by the application of force.

12. The implantation device according to any of claims 1 to 11, **characterized in that** in a last operating position the cannula (10) is received completely within the housing (16), wherein an end position safety device (36) is provided, by means of which the cannula holder (22) can be fixed in the last operating position with respect to the housing (16).

13. The implantation device according to claim 12, **characterized in that** the tappet (32) can be separated from the housing (16) in the second operating position.

14. The implantation device according to any of claims 1 to 13, **characterized in that** the cannula (10) and the tappet (32) have a cross-section deviating from a circular profile.

15. The implantation device according to any of claims 1 to 14, **characterized in that** a stop (42) is provided on a distal end (40) of the housing (16) to restrict the insertion depth.

16. The implantation device according to claim 15, **characterized in that** a beveled contact surface (44) is formed at a predefined insertion angle (WI) on the stop (42).

17. The implantation device according to any of claims 1 to 16, **characterized in that** a viewing area (38) is provided on the housing (16), through which a portion of the cannula (10) protruding from the housing outlet (14) may be viewed.

18. The implantation device according to any of claims 1 to 17, **characterized in that** the tappet (32) is held exchangeably together with the cannula (10).

19. The implantation device according to claim 18, **characterized in that** the cannula holder (22) is configured at least partially as a single-use device, which can be fixed exchangeably on the remaining device.

20. The implantation device according to claim 18, **characterized in that** the second housing part (20) forming the housing outlet (14) is held exchangeably with the cannula (10) on the first housing part (18).

21. The implantation device according to any of claims 18 to 20, **characterized in that** the exchangeable elements can be fixed on the remaining device via a safety coupling, wherein the safety coupling is detachable in the last operating position.

22. The implantation device according to any of claims 18 to 21, **characterized in that**, for reuse of the implantation device (2), the mechanical energy store (30) can be pretensioned with a new exchangeable element by manual relative movement of a housing portion (46), wherein a safety handle (50) is provided, which, in an initial position, blocks the relative movement of the housing portion (46) and, in a release position, is displaceable, in which the relative movement is released.

23. The implantation device according to any of claims 18 to 22, **characterized in that** at least one object (4A; 4B) to be implanted is received in the cannula (10), which is arranged on the distal tip (12) of the cannula (10) and further at least one separate object (4A; 4B) to be implanted is provided, which can be inserted into the cannula (10).

24. The implantation device according to claim 23, **characterized in that** an insertion aid (60) is provided for insertion of the at least one object (4A; 4B) into the cavity (8), said insertion aid being able to be attached on an end of the cannula (10).

25. The implantation device according to claim 23 or 24, **characterized in that** the at least one object (4A; 4B) is received in a sleeve (64), which can be inserted into the cannula (10) .

## Revendications

1. Dispositif d'implantation (2) pour le positionnement d'au moins un objet (4A ; 4B) sous la peau d'un patient
avec un boîtier (16),
un porte-canule (22) déplaçable par rapport au boîtier (16), sur lequel peut être montée une canule (10) avec une pointe distale (12) et une cavité (8) pour la réception du au moins un objet (4A ; 4B) à implanter,
et un poussoir (32) pouvant être fixé par rapport au boîtier (16), qui présente une extrémité distale (34) pouvant être positionnée dans la cavité (8) de la canule (10),
dans lequel la canule (10) est déplaçable d'une première position de piquage par rapport à une sortie de boîtier (14) sur une première voie de course de retour le long du poussoir (32) dans une première position de retrait, afin de déposer un premier objet (4A) reçu dans la cavité (8),
et la canule (10) est déplaçable de la première position de retrait conjointement avec le poussoir (32) dans une deuxième position de piquage par rapport à la sortie de boîtier (14) et
de la deuxième position de piquage dans une deuxième position de retrait le long du poussoir (32) sur une deuxième voie de course de retour, afin de déposer un deuxième objet (4B) reçu dans la cavité (8),
**caractérisé en ce que** le porte-canule (22) pour le déplacement dans la deuxième position de piquage peut être fixé sur une première partie de boîtier (18), qui est reliée fixement à l'extrémité distale (34) du poussoir (32) et est retenue de manière déplaçable dans une deuxième partie de boîtier (20) formant la sortie de boîtier (14).

2. Dispositif d'implantation selon la revendication 1, **caractérisé en ce que** la première partie de boîtier (18) est montée de manière mobile sur la deuxième partie de boîtier (20).

3. Dispositif d'implantation selon la revendication 1 ou 2, **caractérisé en ce que** la deuxième partie de boîtier (20) présente des moyens (45) antidérapants sur une face extérieure.

4. Dispositif d'implantation selon l'une quelconque des revendications 1 à 3, **caractérisé en ce que** des moyens d'arrêt de boîtier (58), qui fixent les deux parties de boîtier (18, 20) l'une sur l'autre jusqu'à l'atteinte de la première position de retrait de la canule (10), sont prévus entre la première partie de boîtier (18) et la deuxième partie de boîtier (20).

5. Dispositif d'implantation selon l'une quelconque des revendications 1 à 4, **caractérisé en ce que** des moyens de rappel sont prévus, au moyen desquels le déplacement de la canule (10) par-dessus au moins une des voies de course de retour peut être exécuté automatiquement, dans lequel les moyens de rappel présentent un accumulateur de force mécanique (30), au moyen duquel le porte-canule (22) est déplaçable.

6. Dispositif d'implantation selon la revendication 5, **caractérisé en ce que** les moyens de rappel présentent une transmission (37), par l'intermédiaire de laquelle le porte-canule (22) est déplaçable.

7. Dispositif d'implantation selon la revendication 5 ou 6, **caractérisé en ce que** les moyens de rappel présentent un système d'amortissement (39), au moyen duquel une vitesse de déplacement pendant le déplacement par-dessus au moins une des voies de course de retour est réglable.

8. Dispositif d'implantation selon l'une quelconque des revendications 5 à 7, **caractérisé en ce que** les moyens de rappel présentent un bouton de déclenchement (26), au moyen duquel le déplacement sur au moins une des voies de course de retour peut être déclenché.

9. Dispositif d'implantation selon la revendication 8, **caractérisé en ce que** des moyens de fixation (24), qui peuvent être reliés de manière détachable à des moyens de fixation complémentaires (25) du porte-canule (22), sont prévus sur le bouton de déclenchement (26).

10. Dispositif d'implantation selon l'une quelconque des revendications 1 à 9, **caractérisé en ce que** le déplacement sur au moins une des voies de course de retour peut être exécuté manuellement.

11. Dispositif d'implantation selon la revendication 10, **caractérisé en ce qu'**un bouton coulissant (52) mobile sur le boîtier (16), qui peut être libéré d'un arrêt par soumission à une force, est prévu pour le déplacement manuel.

12. Dispositif d'implantation selon l'une quelconque des revendications 1 à 11, **caractérisé en ce que** la canule (10) dans une dernière position de retrait est reçue entièrement à l'intérieur du boîtier (16), dans lequel un blocage de fin de course (36) est prévu, au moyen duquel le porte-canule (22) dans la dernière position de retrait peut être fixé par rapport au boîtier (16).

13. Dispositif d'implantation selon la revendication 12, **caractérisé en ce que** le poussoir (32) dans la deuxième position de retrait peut être séparé du boîtier (16).

14. Dispositif d'implantation selon l'une quelconque des revendications 1 à 13, **caractérisé en ce que** la canule (10) et le poussoir (32) présentent une section transversale différant d'un profil circulaire.

15. Dispositif d'implantation selon l'une quelconque des revendications 1 à 14, **caractérisé en ce qu'**une butée (42) pour la limitation de profondeur de piquage est prévue sur une extrémité distale (40) du boîtier (16).

16. Dispositif d'implantation selon la revendication 15, **caractérisé en ce qu'**une surface de placement (44) chanfreinée selon un angle de piquage (WI) prédéfini est réalisée sur la butée (42).

17. Dispositif d'implantation selon l'une quelconque des revendications 1 à 16, **caractérisé en ce qu'**une zone de visualisation (38), à travers laquelle une section de la canule (10) faisant saillie de la sortie de boîtier (14) peut être examinée, est prévue sur le boîtier (16).

18. Dispositif d'implantation selon l'une quelconque des revendications 1 à 17, **caractérisé en ce que** le poussoir (32) est retenu de manière interchangeable conjointement avec la canule (10).

19. Dispositif d'implantation selon la revendication 18, **caractérisé en ce que** le porte-canule (22) est réalisé au moins en partie sous la forme d'un système jetable, qui peut être fixé de manière interchangeable sur le reste du dispositif.

20. Dispositif d'implantation selon la revendication 18, **caractérisé en ce que** la deuxième partie de boîtier (20) formant la sortie de boîtier (14) est retenue sur la première partie de boîtier (18) de manière interchangeable avec la canule (10).

21. Dispositif d'implantation selon l'une quelconque des revendications 18 à 20, **caractérisé en ce que** les éléments interchangeables peuvent être fixés sur le reste du dispositif par l'intermédiaire d'un accouplement d'immobilisation, dans lequel l'accouplement d'immobilisation peut être libéré dans la dernière position de retrait.

22. Dispositif d'implantation selon l'une quelconque des revendications 18 à 21, **caractérisé en ce que** l'accumulateur de force mécanique (30) pour la réutilisation du dispositif d'implantation (2) avec un nouvel élément interchangeable peut être précontraint par un mouvement relatif manuel d'une section de boîtier (46), dans lequel une poignée d'immobilisation (50) est prévue, qui dans une position de départ bloque le mouvement relatif de la section de boîtier (46) et est déplaçable dans une position de déblocage, dans laquelle le mouvement relatif est débloqué.

23. Dispositif d'implantation selon l'une quelconque des revendications 18 à 22, **caractérisé en ce qu'**au moins un objet (4A ; 4B) à implanter, qui est disposé sur la pointe distale (12) de la canule (10), est reçu dans la canule (10) et en outre au moins un objet (4A ; 4B) séparé à implanter, qui peut être introduit dans la canule (10), est prévu.

24. Dispositif d'implantation selon la revendication 23, **caractérisé en ce qu'**un auxiliaire d'introduction (60) pour l'introduction du au moins un objet (4A ; 4B) dans la cavité (8) est prévu, qui peut être monté sur une extrémité de la canule (10).

25. Dispositif d'implantation selon la revendication 23 ou 24, **caractérisé en ce que** le au moins un objet (4A ; 4B) est reçu dans un manchon (64), qui peut être introduit dans la canule (10).
